# DEMANDE DE BREVET EUROPEEN

(11) **EP 1 365 014 A1**
(43) Date de publication de la demande: **26.11.2003**
(21) Numéro de dépôt: 03291204.0
(22) Date de dépôt: 21.05.2003
(51) Int. Cl.: C12M 1/14, C12M 3/04

(54) **Bioréacteur formant une cuve rigide**

(30) Priorité: 21.05.2002 FR 0206146
(71) Demandeur: L'OREAL, 75008 Paris (FR)
(72) Inventeur: Martin, Richard, 37210 Rochecorbon (FR); Hilaire, Pascal, 37210 Vouvray (FR)
(74) Mandataire: Leray, Noelle

(57) **Abrégé**

L'invention propose un bioréacteur (10) pour la culture de cellules animales, végétales, microbiennes ou d'algues ou de leurs co-cultures, du type comportant un corps (12) qui délimite un volume intérieur (14) apte à recevoir un liquide de culture (16) et un volume gazeux (17) au-dessus du liquide de culture (16), et qui comporte des moyens pour l'introduction (18) et/ou l'extraction (20) d'éléments respectivement dans et/ou hors du volume intérieur (14) du corps (12), et du type qui comporte des moyens d'entraînement (22) du corps (12) en mouvement oscillant de sorte à obtenir une agitation du liquide de culture (16), caractérisé en ce que le corps (12) est une cuve rigide.

## Description

L'invention propose un bioréacteur pour la culture de cellules animales, végétales, microbiennes ou d'algues ou de leurs co-cultures, du type comportant un corps qui délimite un volume intérieur apte à recevoir un liquide de culture et un volume gazeux au-dessus du liquide de culture, et qui comporte des moyens pour l'introduction et/ou l'extraction d'éléments respectivement dans et/ou hors du volume intérieur du corps, et du type qui comporte des moyens d'entraînement du corps en mouvement oscillant de sorte à obtenir une agitation du liquide de culture.

Les avancées technologiques dans le domaine des biotechnologies entraînent une augmentation des besoins en cellules animales, végétales, microbiennes ou d'algues de sorte qu'il est nécessaire d'accroître les capacités de production de ces cellules.

La production de cellules s'effectue en les cultivant dans un liquide de culture qui comporte des composants nécessaires à leur croissance et qui peut être mis au contact d'un gaz contenant aussi des composants nécessaires à la croissance des cellules. En particulier, les cellules dites "aérobies" sont mises au contact avec l'oxygène de l'air, qui est un composant nécessaire à leur développement, par des moyens d'injection de l'air dans le liquide de culture.

Le liquide de culture est en outre agité par des moyens d'agitation afin d'optimiser le contact entre les cellules et les composants nécessaires à leur croissance contenus dans le liquide et/ou dans le gaz.

Pour la culture des cellules dans de telles conditions, il a été proposé d'utiliser des cuves en acier inoxydable conforme aux exigences alimentaires, de désignation Z2 CND17.12 (norme NF A02-004) ou 316L (norme AISI), et dans lesquelles le liquide de culture est agité par un agitateur interne, par exemple du type à pales.

La culture de certaines catégories de cellules dites "phototrophes" nécessite un éclairement important du liquide de culture, cependant, les cuves réalisées en acier inoxydable ne permettent d'assurer qu'un éclairement du liquide de culture relativement faible, par exemple si la cuve est munie de hublots, voire nul si elle ne l'est pas.

De plus, l'utilisation d'un agitateur interne provoque un "cisaillement" du liquide de culture qui endommage les cellules et ralentit le développement de celles-ci.

Il a aussi été proposé de cultiver les cellules dans un bioréacteur composé de plusieurs flacons, ou bouteilles, d'axes horizontaux et agencés en un carrousel qui sont mobiles en rotation continue autour d'un axe horizontal, comme décrit et représenté dans le document US-A-6.066.497. Chaque bouteille comporte des moyens pour inoculer ou pour prélever certains produits, respectivement dans ou depuis leur volume intérieur.

Les dimensions des bouteilles sont réduites pour qu'une personne puisse les transporter sans efforts. Ainsi, les bouteilles ont une capacité maximale relativement restreinte. De plus, les bouteilles ne peuvent être utilisées qu'à partir d'une quantité minimale de liquide, de sorte qu'en cours de culture, il est relativement difficile de modifier la quantité de liquide de culture.

Cependant, lorsque l'on désire effectuer une inoculation d'un produit dans le liquide de culture, il faut effectuer une inoculation pour chaque bouteille, ce qui multiplie le risque de contamination du liquide par le nombre de bouteilles.

Enfin, comme décrit dans le document FR-A-2.519.020, il a été proposé de cultiver les cellules dans un bioréacteur qui consiste en un sac de plastique plus ou moins translucide monté sur un plateau animé d'un mouvement de balancier.

Le sac est rempli en partie par le liquide de culture, et le volume du sac est complété par de l'air injecté, de sorte à permettre des échanges gazeux entre le gaz et les cellules. Cependant, l'air qui est introduit dans le bioréacteur doit être stérile, de sorte que le bioréacteur nécessite des moyens complexes de stérilisation de l'air qui sont relativement coûteux.

De plus, la pression exercée par le liquide de culture sur les parois du sac a naturellement tendance à lui faire prendre une forme sensiblement sphérique qui, de manière connue, est la forme géométrique permettant d'avoir une surface extérieure minimale pour un volume intérieur maximal, de sorte que cela réduit la surface libre du liquide de culture qui est en contact avec l'air.

L'invention vise à proposer un bioréacteur permettant d'avoir une plus grande surface libre de contact du liquide de culture avec le gaz présent dans le bioréacteur pour un même volume de liquide de culture.

Dans ce but, l'invention propose un bioréacteur du type décrit précédemment, caractérisé en ce que le corps est une cuve rigide.

Selon d'autres caractéristiques de l'invention :
- le corps peut être réalisé en un matériau perméable à la lumière ;
- le corps peut comporter des moyens pour augmenter la surface du liquide de culture qui est en contact avec le volume gazeux présent dans la partie supérieure du corps ;
- le bioréacteur peut comporter au moins un récipient ouvert vers le haut, qui est agencé à l'intérieur du corps, qui est entraîné en mouvement avec le corps et qui, pour au moins une orientation du corps, est apte à isoler une certaine quantité de liquide de culture du reste du liquide de culture contenu dans le corps ;
le bioréacteur peut comporter au moins un filtre permettant un échange gazeux entre le volume intérieur du corps et l'extérieur, la présence d'un tel filtre permettant des échanges gazeux très importants ce qui favorise la croissance des cellules ;
- la face supérieure du corps peut être ouverte, au moins en partie, et la face supérieure du corps peut être fermée de manière stérile par un élément de fermeture ;
- l'élément de fermeture peut être un film perméable aux gaz de manière à former le filtre ;
- l'élément de fermeture peut être un couvercle qui porte le filtre et les moyens pour l'introduction et/ou l'extraction d'éléments ;
- le corps peut être entraîné en mouvement d'oscillation autour d'un axe sensiblement horizontal ;
- le corps est entraîné en translation alternée parallèlement à une direction sensiblement horizontale.

D'autres caractéristiques et avantages de l'invention apparaîtront à la lecture de la description détaillée qui suit pour la compréhension de laquelle on se reportera aux figures annexées parmi lesquelles :
- la figure 1 est une représentation schématique en perspective d'un bioréacteur conforme aux enseignements de l'invention ;
- la figure 2 est une vue similaire à celle de la figure 1 dans laquelle le corps est entraîné en mouvement alterné de translation horizontale ;
- la figure 3 est une vue de détail à plus grande échelle des moyens de verrouillage de l'élément de fermeture du corps ;
- la figure 4 est une vue de côté du corps représenté à la figure 1, dans laquelle le corps est représenté dans sa position de repos ;
- la figure 5 est une vue similaire à celle de la figure 4 dans laquelle le corps est représenté lors d'une oscillation ;
- la figure 6 est une vue similaire à celle de la figure 4 représentant une variante de réalisation de l'invention dans laquelle le corps comporte des récipients qui s'étendent chacun sensiblement sur toute la longueur du corps ;
- la figure 7 est une vue similaire à celle de la figure 5, conformément à la variante de réalisation de l'invention.

Pour la description de l'invention, on adoptera à titre non limitatif les orientations verticale, longitudinale et transversale selon le repère V, L, T indiqué aux figures.

Dans la description qui va suivre, des éléments identiques, similaires ou analogues seront désignés par les mêmes chiffres de référence.

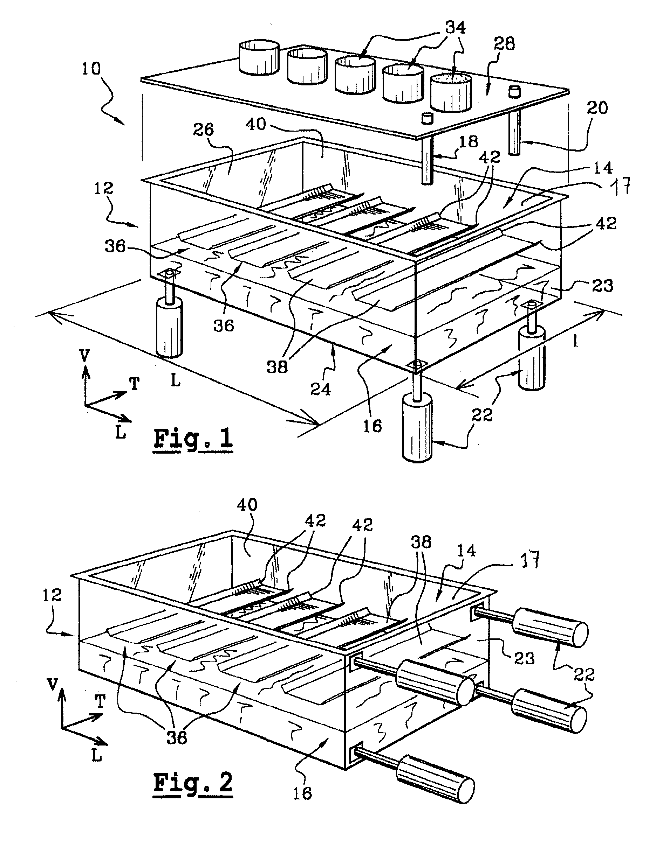

On a représenté à la figure 1 un bioréacteur 10 pour la culture de cellules animales, végétales, microbiennes ou d'algues ou de leurs co-cultures qui comporte un corps 12 réalisé dans un matériau rigide apte à laisser passer la lumière et délimitant, à l'intérieur de la cuve ainsi réalisée, un volume 14 destiné à recevoir un liquide de culture 16.

Le liquide de culture 16 constitue le milieu dans lequel les cellules se développent, et il contient des éléments nutritifs nécessaires à la croissance des cellules.

Un volume de gaz 17, par exemple d'air dans le cas où les cellules cultivées sont des cellules "aérobies", est présent au-dessus du liquide de culture 16.

Un paramètre qui influence la performance du bioréacteur 10 est la concentration des cellules dans le liquide de culture 16 qui doit être comprise dans une plage de valeurs donnée. Puisque le but du bioréacteur 10 est de produire des cellules, il est nécessaire de renouveler et/ou de compléter le liquide de culture avec du liquide de culture neuf au fur et à mesure de leur multiplication.

A cet effet, le bioréacteur 10 comporte des moyens d'introduction 18 d'éléments dans le volume intérieur 14 du corps 12, notamment de liquide de culture neuf qui sont conçus pour que l'introduction du liquide de culture neuf s'effectue sans introduction d'éléments étrangers polluants dans le volume intérieur 14 du corps.

Le bioréacteur 10 comporte aussi des moyens 20 pour l'extraction d'éléments du volume intérieur 14 du corps 12, qui sont notamment utilisés pour le prélèvement d'une faible quantité de liquide de culture 16 en vue d'une analyse qui permettra de vérifier le bon déroulement de la culture des cellules.

Le corps 12 est animé d'un mouvement alternatif permettant d'avoir un mélange continu ou périodique du liquide de culture 16. Ce mélange du liquide de culture 16 permet d'assurer les échanges gazeux entre les cellules et le gaz 17 présent au-dessus du liquide, notamment avec l'oxygène contenu dans l'air lorsqu'il s'agit de la culture de cellules dites "aérobies". De plus, le mélange du liquide de culture 16 permet d'optimiser le contact entre les cellules et les éléments nutritifs contenus dans le liquide de culture 16. Un tel système d'agitation externe permet d'éviter tout cisaillement du liquide de culture.

Lorsque le corps 12 est animé du mouvement alternatif la présence du volume gazeux au-dessus du liquide de culture 16 permet de former des turbulences, qui se traduisent par la formation de vagues (non représentées). Il résulte de ces turbulences que les cellules sont animées d'un mouvement ou brassage dans le liquide de culture, dont au moins une composante est verticale, et donc une modification continue des cellules qui sont à la surface 16s du liquide de culture 16, augmentant de ce fait les échanges gazeux.

Comme représenté à la figure 1, le bioréacteur comporte un ensemble de vérins 22, agencés ici au-dessous du corps 12, qui l'entraînent en mouvement d'oscillation autour d'un axe transversal horizontal.

Le mouvement d'oscillation du corps 12 peut être obtenu par tout autre moyen connu de l'homme du métier, par exemple, comme décrit dans le document WO-A-00.66706, le bioréacteur comporte un plateau oscillant sur lequel est monté le corps.

Selon une variante représentée à la figure 2, le corps ou cuve 12 est entraîné en translation alternée parallèlement à une direction longitudinale horizontale. Les vérins sont alors orientés parallèlement à la direction du mouvement, c'est-à-dire parallèlement à la direction longitudinale, et ils agissent sur une paroi latérale verticale 23 du corps 12.

Afin d'améliorer le rendement du bioréacteur, la surface supérieure libre 16s du liquide 16 qui est en contact avec les gaz 17 contenus dans le volume intérieur 14 du corps 12 doit être la plus importante possible de manière à augmenter le volume des échanges gazeux naturels entre le liquide de culture 16 et le volume gazeux 17, et aussi d'autoriser un mouvement du liquide permettant un brassage de celui-ci.

A cet effet, et conformément à l'invention, le corps 12 est une cuve rigide, ici de forme parallélépipédique rectangle, de sorte que lorsqu'il est dans une position de repos, il comporte un fond rigide 24 horizontal d'orientation générale longitudinale et des parois latérales verticales 23 rigides.

Selon une variante de réalisation non représentée, le corps 12 est constitué d'une armature rigide. Le fond 24 et les parois latérales 23 sont réalisées en un matériau souple et ils sont maintenus en forme par l'armature.

Puisque le corps 12 est rigide, la surface 16s du liquide de culture 16 est sensiblement constante pour une position donnée de la cuve, quel que soit le volume de liquide de culture 16 présent à l'intérieur du corps 12, contrairement aux bioréacteurs dont le corps est un sac souple dont les parois se déforment sous la pression exercée par le fluide de sorte que la surface du liquide de culture qui est en contact avec les gaz est alors réduite.

Pour favoriser la croissance des cellules dites "phototrophes", qui ont besoin d'une grande quantité de lumière pour pouvoir se développer, le corps 12 est avantageusement réalisé en un matériau rigide qui est perméable à la lumière. Ce matériau est de préférence un polymère transparent tel que le polycarbonate.

Un avantage du polycarbonate est qu'il résiste à des températures pouvant atteindre environ 135°C, de sorte que le corps 12 peut être stérilisé dans un autoclave.

La stérilisation du corps 12 est alors grandement simplifiée si on la compare à la stérilisation des bioréacteurs pour lesquels elle est réalisée à la vapeur et sur place, en nécessitant l'utilisation de moyens complexes et coûteux.

La face supérieure 26 du corps 12 est ouverte et elle permet le passage des moyens d'introduction 18 et des moyens d'extraction 20. Cependant, la culture des cellules nécessite une absence rigoureuse de cellules étrangères de sorte que la face supérieure 26 doit être obturée de manière à garantir la stérilité du bioréacteur 10.

A cet effet, le bioréacteur 10 comporte un élément de fermeture 28 qui recouvre la face supérieure 26 de sorte que le volume intérieur 14 du corps 12 soit protégé de toute contamination extérieure.

Pour que la fermeture de la face supérieure 26 soit étanche et donc stérile, le bioréacteur 10 comporte un joint 30 qui est interposé entre l'élément de fermeture 28 et le corps 12, et qui est comprimé par des moyens de verrouillage 32. Ces moyens de verrouillage 32, qui consistent ici en un système vis-écrou, permettent de réaliser le serrage ou compression du joint 30 et le verrouillage en position de l'élément de fermeture 28 et ils sont agencés à l'extérieur du corps 12 afin que leur manipulation n'entraîne pas de contamination.

Du fait des échanges gazeux entre les cellules et le volume de gaz 17, il est nécessaire de renouveler constamment ou régulièrement le gaz 17 qui est présent dans le volume intérieur 14 du corps 12.

Le renouvellement du gaz est obtenu grâce à des filtres 34, par exemple par une membrane microporeuse qui ne laisse passer que des molécules et atomes contenus dans le gaz et qui empêchent le passage de toute autre cellule qui pourrait contaminer le liquide de culture 16.

De tels filtres permettent une aération dite "passive" qui ne perturbe pas les équilibres gazeux à l'intérieur du bioréacteur 10, contrairement aux systèmes d'injection d'air utilisés dans les bioréacteurs traditionnels. Ainsi, les gaz que produisent les cellules au démarrage de la culture et qui favorisent le développement des cellules, ne sont pas éliminés alors qu'ils le seraient par un système de propulsion de gaz.

Selon un premier mode de réalisation représenté schématiquement aux figures, l'élément de fermeture 28 est un couvercle rigide qui porte les moyens d'introduction 18 et les moyens d'extraction 20 et qui porte plusieurs filtres 34.

Selon un deuxième mode de réalisation, non représenté, l'élément de fermeture 28 est un film ou une membrane qui recouvre entièrement la face supérieure 26 du corps et qui est réalisé de sorte à avoir les mêmes caractéristiques que les filtres 34, c'est-à-dire de ne laisser passer que les molécules et atomes contenus dans le gaz, tout en empêchant le passage d'éléments pouvant contaminer le liquide de culture 16.

L'aire de la surface 16s du liquide de culture 16 est limitée par les dimensions du corps, c'est à dire ici par sa longueur "L" et sa largeur "ℓ".

Pour augmenter la surface de liquide de culture 16 qui est en contact avec le gaz, et selon une variante de réalisation de l'invention, le corps 12 comporte plusieurs récipients 36 qui consistent ici en des éléments concaves ouverts vers le haut en forme générale de vasque.

Chaque récipient comporte un plateau transversal horizontal 38 qui relie les deux parois longitudinales verticales 40 du corps 12 et dont les bords d'extrémité transversale 42 sont inclinés vers le haut.

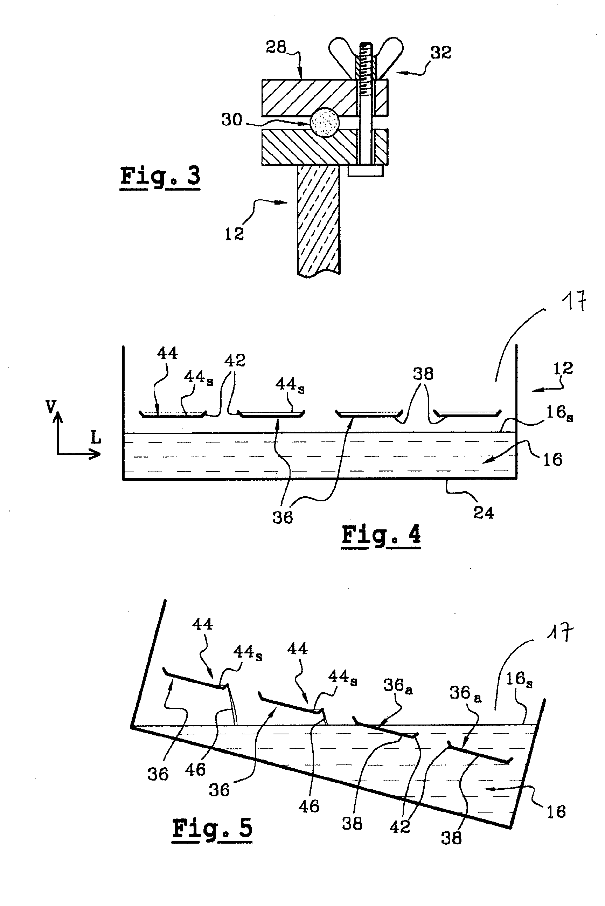

Les récipients 36 sont agencés de sorte qu'ils s'étendent au-dessus du liquide de culture 16 lorsque le corps 12 est dans sa positon de repos représentée à la figure 4. Ils sont aussi agencés pour qu'au moins une partie des récipients 36 soit immergée dans le liquide de culture 16 dans au moins une position du corps 12 autre que sa position de repos, notamment lors, du mouvement d'oscillation du corps, comme on l'a représenté à la figure 5.

Ainsi, dans un premier mouvement du corps représenté à la figure 5, des premiers récipients 36a sont immergés dans le liquide. Lorsque le corps 12 pivote autour de son axe d'oscillation pour revenir vers sa position de repos, ces premiers récipients 36a ont chacun prélevé une certaine quantité de liquide de culture 44 et ils l'isolent alors du reste du liquide de culture 16.

Les échanges gazeux s'effectuent ainsi au niveau de la surface 16s du liquide de culture, et au niveau de la surface 44s de la quantité de liquide prélevée 44 stockée provisoirement dans chaque récipient 36. La surface d'échange totale est donc augmentée.

Lorsque le corps 12 bascule vers la position opposée à celle représentée à la figure 5, c'est-à-dire qu'il est incliné par rapport à sa position de repos, et que les premiers récipients 36a s'étendent au-dessus du liquide de culture 16, une partie 46 de la quantité de liquide prélevée 44 se déverse des premiers récipients 36a vers le reste du liquide de culture 16.

Cette quantité 46 qui se déverse permet d'augmenter la surface totale du liquide de culture du fait de sa propre surface.

Lorsque le corps 12 revient dans sa position pour laquelle les premiers récipients 36a sont immergés, la quantité de liquide de culture prélevé 44 est réintroduite et se mélange dans le reste du liquide de culture 16.

Combinée au brassage du liquide de culture 16, l'action des récipients 36 permet d'augmenter la surface d'échange gazeux du liquide de culture 16.

Selon une variante non représentée de l'invention, le corps 12 comporte plusieurs séries de récipients 36 qui sont agencées à différentes distances du fond 24 de sorte qu'au moins une partie des récipients 36 soit efficace quelle que soit la hauteur du liquide de culture 16 contenu dans le corps 16.

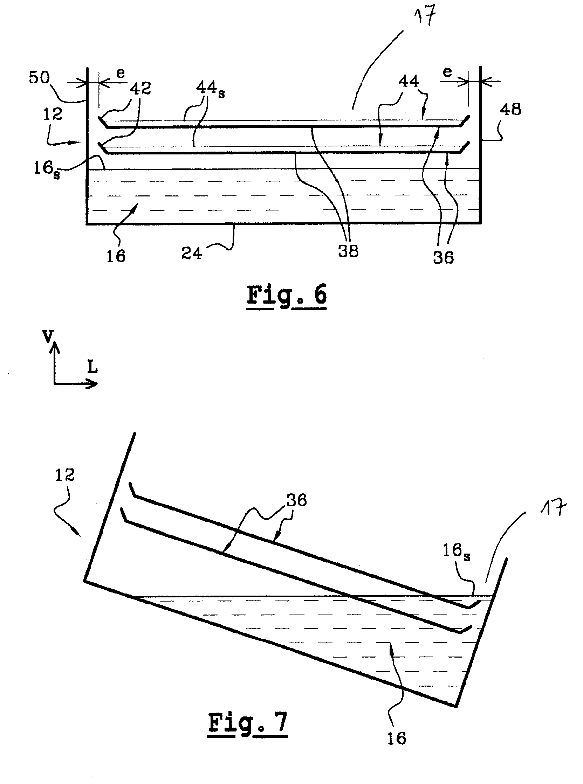

Selon une variante de réalisation de l'invention représentée aux figures 6 et 7, le corps 12 comporte plusieurs récipients 36 répartis à différentes distances du fond 24, et chaque récipient 36 est agencé à une distance du fond 24 différente des autres récipients 36.

La longueur de chaque récipient 36 est sensiblement inférieure à la longueur "L" du corps 12 de sorte que la surface de la quantité de liquide de culture prélevée 44 soit la plus grande possible, tout en laissant un espace "e" entre le récipient 36 et les parois transversales avant 48 et arrière 50.

Avantageusement, pour la culture de cellules "phototrophes", les récipients 36 sont réalisés dans le même matériau transparent que le corps, de sorte qu'ils ne réduisent pas l'éclairement de la totalité des cellules.

Le bioréacteur 10 permet de rajouter du liquide de culture neuf dans le liquide de culture 16, sans qu'il soit nécessaire d'interrompre la culture des cellules. Ainsi, le niveau global du liquide de culture 16 à l'intérieur du corps 12 augmentera pour chaque ajout de liquide de culture neuf.

Il sera aussi compris que des inversions mécaniques simples peuvent constituer des variantes de réalisation de l'invention. Par exemple, les moyens d'introduction 18 et les moyens d'extraction 20 peuvent être portés par une paroi verticale du corps 12. Le bioréacteur peut également avoir tout autre type de mouvement oscillant, par exemple un mouvement qui oscille autour d'un axe autre qu'un axe central à la cuve, notamment un axe formé selon un des côtés de la cuve lorsque celle-ci est rectangulaire.

Un bioréacteur conforme à l'invention permet ainsi d'avoir, au début de la culture, une faible quantité de cellules, par exemple 1 litre, qui est transférée dans un volume de milieu approprié, par exemple 10 litres. Ensuite, au fur et à mesure de la croissance des cellules, on ajoute du liquide de culture neuf, jusqu'à atteindre la capacité maximale du bioréacteur, par exemple 100 litres, sans avoir à transvaser le liquide de culture d'un bioréacteur vers un autre, en limitant de ce fait les risques de contaminations. Le bioréacteur selon l'invention permet des échanges gazeux très importants ce qui favorise la croissance des cellules.

## Revendications

1. Bioréacteur (10) pour la culture de cellules animales, végétales, microbiennes ou d'algues ou de leurs co-cultures, du type comportant un corps (12) qui délimite un volume intérieur (14) apte à recevoir un liquide de culture (16) et un volume gazeux (17) au-dessus du liquide de culture (16), et qui comporte des moyens pour l'introduction (18) et/ou l'extraction (20) d'éléments respectivement dans et/ou hors du volume intérieur (14) du corps (12), et du type qui comporte des moyens d'entraînement (22) du corps (12) en mouvement oscillant de sorte à obtenir une agitation du liquide de culture (16),
**caractérisé en ce que** le corps (12) est une cuve rigide.

2. Bioréacteur (10) selon la revendication précédente, **caractérisé en ce que** le corps (12) est réalisé en un matériau perméable à la lumière.

3. Bioréacteur (10) selon l'une des revendications précédentes, **caractérisé en ce que** le corps (12) comporte des moyens (36) pour augmenter la surface du liquide de culture (16) qui est en contact avec le volume gazeux (17) présent dans la partie supérieure du corps (12).

4. Bioréacteur (10) selon la revendication précédente, **caractérisé en ce qu'**il comporte au moins un récipient (36) ouvert vers le haut, qui est agencé à l'intérieur du corps (12), qui est entraîné en mouvement avec le corps (12) et qui, pour au moins une orientation du corps (12), est apte à isoler une certaine quantité de liquide de culture (44) du reste du liquide de culture (16) contenu dans le corps (12).

5. Bioréacteur (10) selon l'une des revendications précédentes, **caractérisé en ce qu'**il comporte au moins un filtre (34) permettant un échange gazeux entre le volume intérieur (14) du corps (12) et l'extérieur.

6. Bioréacteur (10) selon l'une quelconque des revendications précédentes, **caractérisé en ce que** la face supérieure (26) du corps (12) est ouverte, au moins en partie, et **en ce que** la face supérieure (26) du corps (12) est fermée de manière stérile par un élément de fermeture (28).

7. Bioréacteur (10) selon la revendication précédente, en combinaison avec la revendication 5, **caractérisé en ce que** l'élément de fermeture (28) est un film perméable aux gaz de manière à former le filtre.

8. Bioréacteur (10) selon la revendication 6, **caractérisé en ce que** l'élément de fermeture (28) est un couvercle qui porte le filtre (34) et les moyens pour l'introduction (18) et/ou l'extraction (20) d'éléments.

9. Bioréacteur (10) selon l'une quelconque des revendications précédentes, **caractérisé en ce que** le corps (12) est entraîné en mouvement d'oscillation autour d'un axe sensiblement horizontal.

10. Bioréacteur (10) selon l'une quelconque des revendications 1 à 8, **caractérisé en ce que** le corps (12) est entraîné en translation alternée parallèlement à une direction sensiblement horizontale.
